# EUROPEAN PATENT SPECIFICATION

(11) **EP 0 832 980 B1**
(45) Date of publication and mention of the grant of the patent: **19.06.2002**
(21) Application number: 97113730.2
(22) Date of filing: 22.01.1990
(51) Int. Cl.: C12N 15/85, A61K 39/12, C07H 15/12, C12N 15/00, C12N 5/10, C12N 7/00, A61K 48/00

(54) **Recombinant therapies for infection and hyperproliferative disorders**
Rekombinanttherapien für Infektionen und hyperproliferative Störungen
Thérapie recombinante des infections et désordres hyperprolifératifs

(30) Priority: 23.01.1989 US 300637; 17.01.1990 US 461461
(43) Date of publication of application: 01.04.1998
(62) Divisional of application: 90902891.2
(73) Proprietor: CHIRON CORPORATION, Emeryville, California 94608 (US)
(72) Inventor: Goldsmith, Mark A., San Francisco, California 94114 (US); Ralston, Robert O., San Francisco, CA 94122 (US)
(74) Representative: Irvine, Jonquil Claire

(56) References cited:
- EP-A- 0 334 301
- WO-A-91/10453
- US-A- 4 738 922
- MUESING, M.A. ET AL.: "Regulation of mRNA accumulation by a Human Immunodeficiency Virus trans-activator protein" CELL, vol. 48, 27 February 1987, NA US, pages 691-701, XP002050787
- FELBER B K ET AL: "A QUANTITATIVE BIOASSAY FOR HIV-1 BASED ON TRANS-ACTIVATION" SCIENCE., vol. 239, no. 4835, 1 January 1988, LANCASTER, PA US, pages 184-187, XP000212648
- HEYMAN R A ET AL: "THYMIDINE KINASE OBLITERATION: CREATION OF TRANSGENIC MICE WITH CONTROLLED IMMUNE DEFICIENCY" PROCEEDINGS OF THE NATIONAL ACADEMY OF SCIENCES OF USA, vol. 86, no. 8, 1 April 1989, pages 2698-2702, XP000310583
- PATARCA, R. ET AL.: "rpt-1, an intracellular protein from helper/inducer T cells that regulates gene expression of interleukin 2 receptor and human immunodeficiency virus type 1" PROCEEDINGS OF THE NATIONAL ACADEMY OF SCIENCES OF USA., vol. 85, April 1988, WASHINGTON US, pages 2733-2737, XP002050788

## Description

This invention relates to genetic engineering and the treatment of hyperproliferative disorders and infection.

### Background of the Invention

The therapy of viral infection is in its infancy. Bacterial infection is typically treated with agents, such as antibiotics, which take advantage of the differences in metabolism between the infecting organism and its host. However, viruses largely employ the host's own enzymes to effect their replication, and thus leave few opportunities for pharmacological intervention. By employing strong regulatory elements, the virus obtains transcription and translation of its own genes at the expense of host genes.

In mammals, viral infection is combatted naturally by cytotoxic T-lymphocytes, which recognize viral proteins when expressed on the surface of host cells, and lyse the infected cells. Destruction of the infected cell prevents the further replication of virus. Other defenses include the expression of interferon, which inhibits protein synthesis and viral budding, and expression of antibodies, which remove free viral particles from body fluids. However, induction of these natural mechanisms require exposure of the viral proteins to the immune system. Many viruses, for example herpes simplex virus-1 (HSV-1), exhibit a dormant or latent phase, during which little or no protein synthesis is conducted. The viral infection is essentially invisible to the immune system during such phases.

Retroviruses carry the infectious form of their genome in the form of a strand of RNA. Upon infection, the RNA genome is reverse-transcribed into DNA, and is typically then integrated into the host's chromosomal DNA at a random site. On occasion, integration occurs at a site which truncates a gene encoding an essential cellular receptor or growth factor, or which places such a gene under control of the strong viral cis-acting regulatory element, which may result in transformation of the cell into a malignant state.

Viruses may also be oncogenic due to the action of their trans-acting regulatory factors on host cell regulatory sequences. In fact, oncogenesis was the characteristic which lead to the discovery of the first known retroviruses to infect humans. HTLV-I and HTLV-II (human T-lymphotropic viruses I and II) were identified in the blood cells of patients suffering from adult T-cell leukemia (ATL), and are believed to induce neoplastic transformation by the action of their trans activating factors on lymphocyte promoter regions. HTLV-I and II preferentially infect human lymphocytes, and on occasion, cause their transformation into malignancy. Since then, two additional retroviruses have been found to infect humans: HIV-I and HIV-II, the etio-logical agents of AIDS. However, HIV-I and II apparently contribute to cancer only through their immunosuppressive effects.

HIV I and II apparently infect cells which express the CD4 surface protein. This protein is present in abundance on thymocytes and some T-lymphocytes, and to a lesser extent, on some antigen-presenting cells. HIV infection is initially characterized by flu-like symptoms, followed by a long latency period, which may last five to ten years. Upon entering its active phase, HIV infection results in a rapid decline in the population of "helper" T-lymphocytes (T_{H}), which is usually recognized as a decline in the ratio of T4⁺/T8⁺ (CD4⁺/CD8⁺) T-lymphocytes. The patient typically experiences severe diarrhea, and if the central nervous system is infected, exhibits a form of dementia. The depletion of T_{H} cells cripples the immune system, and the patient succumbs to an opportunistic infection by, for example P. carinii or cytomegalovirus, or to Karposi's sarcoma. The natural immune system appears wholly incapable of combatting HIV infection, despite the typical presence of apparently neutralizing serum antibody titers during latency.

Current therapy for HIV infection *per se* is limited mainly to administration of AZT to inhibit viral progression, although M.S. Hirsch, J Infect Dis (1988) 157:427-31 reported synergistic inhibition of HIV by AZT with GM-CSF (granulocyte-monocyte colony stimulating factor) or α interferon. AZT (3'-azido-3'-deoxythymidine) is representative of a class of dideoxynucleoside (ddN) antiviral agents. These agents rely on the ability of host DNA polymerases to reject a ddN, and the tendency of viral polymerases to accept ddNs and incorporate them into replicating polynucleotides. Upon incorporation, a ddN stops polymerization, as it lacks the 3' hydroxyl group necessary for the next phosphodiester linkage. The ddNs are typically inactive in their administered form, and depend on phosphorylation by host cell enzymes for conversion to the active triphosphate ddNTP.

H. Mitsuya et al, Nature (1987) 325:773-78 disclosed the organization of the HIV genome, and suggested various strategies for development of HIV therapies. Speculated therapies included administration of antisense RNA (as free strands or encoded by an "antivirus") to inhibit viral transcription, administration of glycosylation inhibitors, administration of interferons to inhibit viral budding, and administration of dideoxynucleoside analogues to inhibit viral replication. Dideoxynucleoside analog drugs useful for HIV therapy (e.g., AZT, ddC, etc.) depend on host cell enzymes for activation by phosphorylation. D. Baltimore, Nature (1988) 335:395-96 suggested treating AIDS by removing bone marrow cells from an infected subject, and transfecting hematopoietic cells in the bone marrow extract with DNA or virus encoding an RNA or protein able to interfere with HIV growth. The DNA could encode RNA that might bind to HIV regulatory proteins, antisense RNA, mutant viral polypeptides, or a viral DNA-binding protein lacking its regulator function. The transfected cells would then be reintroduced into the subject, and provided with a selective advantage to insure dissemination.

A.I. Dayton et al, Cell (1986) 44:941-47 disclosed that the HIV tat gene is essential for viral protein synthesis and replication. Dayton suggested that one might inhibit HIV by interference with tat, without otherwise affecting the host cell. M.A. Muesing et al, Cell (1987) 48:691-701 disclosed that the tat protein (rather than tat mRNA alone) is essential for transactivation. Muesing also found that a tat-art fusion (having 114 amino acids fused to the C-terminus of tat by deletion of 7 nucleotides) retained full tat activity. A.D. Frankel et al, Science (1988) 240:70-73 disclosed that tat forms metal-linked dimers in *vitro,* and suggested that possible treatments for AIDS may involve chelation of metal ions, or competition for tat monomer binding. A.D. Frankel et al, Proc Nat Acad Sci USA (1988) 85:6297-30 disclosed the synthesis of an HIV-1 tat fragment which retains the metal-binding properties of tat. The fragment formed heterodimers with native tat, and can displace tat in homodimers. Frankel suggested using the tat fragment to inhibit tat dimerization, using liposomes to deliver the peptides or a tat-fragment gene. The amino acid sequence reported for tat from one HIV-1 isolate is 
Met Glu Pro Val Asp Pro Arg Leu Glu Pro
Trp Lys His Pro Gly Ser Gln Pro Lys Thr
Ala Cys Thr Asn Cys Tyr Cys Lys Lys Cys
Cys Phe His Cys Gln Val Cys Phe Ile Thr
Lys Ala Leu Gly Ile Ser Tyr Gly Arg Lys
Lys Arg Arg Gln Arg Arg Arg Pro Pro Gln
Gly Ser Gln Thr His Gln Val Ser Leu Ser
Lys Gln Pro Thr Ser Gln Ser Arg Gly Asp
Pro Thr Gly Pro Lys Glu.
 A similar protein is found in HIV-2.

The tar sequence, which acts in cis and is regulated by HIV tat, is found approximately at nucleotides +19 to +82 in the HIV-1 genome. The sequence contains two extended, inverted repeats, and is thus predicted to be able to form stem loop structures (Muessing, *supra).* HIV-2 exhibits a similar region.

Haseltine, US 4,738,922 disclosed the HTLV I and II LTR promoter regions, and their use with trans-activators (luk) in vectors for amplified expression of heterologous proteins, exemplified by chloramphenicol acetyltransferase (CAT). HTLV-I LTR apparently promotes constitutive expression, which is further amplified in the presence of HTLV-I luk, while HTLV-II LTR apparently requires HTLV-II luk for expression. Haseltine mentioned that viral trans-acting factors can alter the expression of host cell genes as well as viral genes, and Haseltine disclosed the concept of inserting a vector having the HTLV LTR fused to a heterologous gene into a cell having the HTLV genome, which results in the trans-activation of the heterologous gene and the overexpression of its product. Haseltine disclosed the use of the HTLV LTR in the absence of luk to generate empty capsid vaccines, and suggested using the HTLV LTR for expression of antigens, to provide for destruction of the cell by monoclonal or polyclonal antibodies.

E.A. Dzierzak et al, Nature (1988) 331:35-41 disclosed a prototypical gene therapy method in mice. Dzierzak removed bone marrow cells from mice, exposed the mice to lethal radiation, and introduced human β-globin (BG) genes into the removed marrow using a retroviral vector, pSV(X)neo. The BG gene was inserted to read in the opposite direction from proviral transcription, and constructs both with and without the viral LTR enhancer regions were prepared. The mice were then reconstituted with the recombinant bone marrow, containing hematopoietic stem cells. Dzierzak found that human β-globin was expressed in the resulting recombinant mice, and that the expression was found only in cells of erythroid lineage. J-K Yee et al, Proc Nat Acad Sci USA (1987) 84:5197-201 disclosed construction of retroviral vectors encoding HPRT under control of either the metallothionein promoter or the human cytomegalovirus (hCMV) promoter. Yee found that HPRT expression doubled when transcriptional regulatory sequences were deleted from the U3 region of the retroviral LTR.

S.-F. Yu et al, Proc Nat Acad Sci USA (1986) 83:3194-98 disclosed the construction of self-inactivating ("SIN") retroviral gene transfer vectors. SIN vectors are created by deleting the promoter and enhancer sequences from the U3 region of the 3' LTR. A functional U3 region in the 5' LTR permits expression of the recombinant viral genome in appropriate packaging cell lines. However, upon expression of its genomic RNA and reverse transcription into cDNA, the U3 region of the 5' LTR of the original provirus is deleted, and is replaced with the U3 region of the 3' LTR. Thus, when the SIN vector integrates, the non-functional 3' LTR U3 region replaces the functional 5' LTR U3 region, and renders the virus incapable of expressing the full-length genomic transcript. Yu constructed a recombinant virus using the Mo-MuLV LTR regions and packaging (psi) sequence, and inserted a neomycin resistance (Neo) gene under control of either a metallothionein promoter (virus MT-N), an HSV tk promoter (virus TK-N), or a SV40 promoter (virus SV-N) as a selectable marker. Yu also inserted a human c-fos gene under control of a human metallothionein promoter (hMT) into TK-N, and demonstrated inducible transcription of c-fos in NIH 3T3 cells after infection with the recombinant virus.

S.L. Mansour et al, Nature (1988) 336:348-52 disclosed a method for selecting cells after homologous recombination with a linear transfecting vector. A linear vector was prepared having a region homologous to a target gene, a neomycin resistance gene inserted in an exon of the homologous region, and an HSV-tk gene outside the homologous region. Upon specific homologous recombination, the transformed cell displays a phenotype negative for the target region and HSV-tk, and positive for neomycin resistance (homologous recombination into the target site disrupts the target site gene, and fails to incorporate the tk gene). The phenotype distinguishes cells having homologous recombination from non-specific integration, as the latter cells will display a phenotype positive for the target gene, HSV-tk, and neomycin resistance. Neomycin resistance is tested by culturing cells in neomycin, while tk⁺ is tested by culturing cells in gancyclovir (which is converted to a toxic product by tk).

R.D. Palmiter et al, Cell (1987) 80:435-43 disclosed the preparation of transgenic mice having a DNA construct encoding the diphtheria A chain under control of the elastase promoter. The elastase promoter is active only in pancreatic acinar cells, and promotes the expression of elastase. The DNA constructs were microinjected into mouse eggs, and the resulting progeny examined. Transgenic mice in which the construct was active failed to develop normal pancreatic tissue.

M.E. Selsted et al, J Clin Invest (1985) 76:1436-39 disclosed the primary amino acid sequence for three related human cytotoxic effector polypeptides, termed human neutrophil antimicrobial peptides (HNPs). The three HNPs have 29-30 amino acid residues and exhibit activity against bacteria, fungi, and herpes simplex virus (T. Gantz et al, J Clin Invest (1985) 76:1427-35).

T.L. Wasmoen et al, J Biol Chem (1988) 263:12559-63 disclosed the primary amino acid sequence of human eosinophil granule major basic protein (MBP). MBP is an effector polypeptide having 117 amino acid residues, a pI of 10.9, and exhibiting cytotoxic activity against mammalian cells and parasites.

M.A. Adam et al, J Virol (1988) 62:3802-06 disclosed retroviral vectors which exhibit efficient RNA packaging, having a psi+ sequence. R.D. Cone et al, Mol Cell Biol (1987) 7:887-97 disclosed the construction of retroviral vectors (pSVX) including a human β-globin gene, and the use of the recombinant vectors to obtain human β-globin expression in a murine erythroleukemia cell line. A.D. Miller et al, Mol Cell Biol (1986) 6:2895-902 disclosed cell lines useful for packaging replication-defective retroviral vectors.

Guild et al, J Virol (1988) 62:3795-801 disclosed retroviral vectors using the Mo-MuLV LTRs and psi (packaging) sequence, useful for transfer of entire genes into mammalian cells. Guild employed β-actin and histone promoters (which are active in essentially all cells) to obtain transcription of neo^{r} (as a selectable marker). Expression of the neo^{r} was demonstrated *in vivo,* after virus-infected bone marrow cells were used to reconstitute lethally-irradiated mice.

A.D. Friedman et al, Nature (1988) 335:452-54 disclosed transfection of tk⁻ mouse cells with plasmids expressing HSV-1 tk, and HSV-1 VP16. VP16 acts as a trans activator for transcription of the immediate early genes in herpes simplex virus (HSV-1). On the VP16 plasmid, the promoter was replaced with the Mo-MSV promoter, and the carboxy terminal of the VP16 was deleted. The resulting plasmid codes for a mutant VP16 protein which competes with wild type VP16 for DNA binding. Transfected cells exhibited resistance to HSV-1 replication upon later infection. Friedman suggested that one might induce resistance to HIV by transfecting with a dominant mutant of the HIV transactivator protein (tat).

J. Sodroski et al, Science (1985) 229:74-77 disclosed the location of the HIV tat gene. J. Sodroski et al, Science (1985) 227:171-73 disclosed construction of a plasmid having CAT under control of the HIV LTR. The HIV LTR contains the transactivating region (tar) gene which is induced by tat. Sodroski discovered that transactivating factors would induce transcription of genes under control of the HIV LTR. B.M. Peterlin et al, Proc Nat Acad Sci USA (1986) 83:9734-38 disclosed plasmids having the HIV tar gene, and the effects of orientation and position of tar on transactivation by tat. Peterlin found that tar functions best when downstream from a promoter and an enhancer. Activation with tat increased transcription to RNA. G.J. Nabel et al, Science (1988) 239:1299-302 disclosed that a HIV tat-III-CAT fusion plasmid could be activated by HSV and adenovirus trans-acting factors.

B.K. Felber et al, Science (1988) 239:184-87 disclosed an assay for HIV, using CD4-expressing cell lines transfected with the chloramphenicol acetyltransferase (CAT) gene under control of the HIV LTR. Upon infection with HIV, the transfected cell lines expressed CAT in proportion to the amount of virus present. Felber suggested using the assay as a means for screening possible anti-HIV drugs for their ability to inhibit viral growth, and for identifying anti-tat drugs.

EP-A-0340301, which was published on 29th September 1989 but has an earlier priority date and therefore represents prior art under Article 54(3) EPC, discloses retroviral vectors for therapeutic treatment, including such vectors capable of directing expression of an enzyme which renders the cell susceptible to an additional agent.

### Disclosure of the Invention

One aspect of the invention is a method for treating host cells for an infection or a hyperproliferative disorder which is characterized by expression of regulatory factors capable of regulating transcription of DNA, by inserting into the cells a polynucleotide construct having a regulatory region which is activated by the regulatory factor, and an effector gene under control of the regulatory region which renders said cell susceptible to protection or destruction.

In particular, the present invention provides a polynucleotide construct for treating a host cell for a hyperproliferative disorder or infection by an infectious agent, said infection or hyperproliferative disorder being characterised by a human disease-associated transacting regulatory factor capable of regulating expression of genes, which construct comprises:
(i) at least two tandem copies of a cis-acting regulatory sequence which are controllable by said trans-acting factor; and
(ii) an effector gene under the control of said cis-acting regulatory sequence, said effector gene encoding an enzyme which renders said cell susceptible to an additional agent.

Suitable effector genes for use in such a construct may encode enzymes which activate anti-viral compounds, and the like. For example, the activation region may contain at least two tandem copies of a cis-acting regulatory sequence homologous to the HIV tar region, and the effector gene may encode HSV-1 thymidine kinase (HSV-1 tk). In the case of cells infected with HIV and also carrying such a construct, the HIV tat protein activates the tar region, and induces transcription and expression of HSV-1 tk resulting in cytotoxicity when treated with dideoxynucleoside agents such as gancyclovir.

Another aspect of the invention is a composition useful for delivering the DNA constructs of the invention to host cells.

Another aspect of the invention is a method for protecting specific cell populations within an organism from viral infection, by inserting into the cells of the population a polynucleotide construct of the invention.

### Brief Description of the Drawings

Figure 1 is a diagram of a generic polynucleotide construct of the invention.
Figure 2 is a diagram of the vector pTB1.
Figure 3 is a diagram of the vector pMXSVNeotar/tk.
Figure 4 graphically illustrates the results of the experiment described in Example 5.
Figure 5 graphically illustrates the results of the experiment described in Example 4.

### Modes of Carrying Out The Invention

### A. Definitions

The term "treatment" as used herein refers to reducing or alleviating symptoms in a subject, preventing symptoms from worsening or progressing, inhibition or elimination of the causative agent, or prevention of the infection or disorder in a subject who is free therefrom. Thus, for example, treatment of a cancer patient may be reduction of tumor size, elimination of malignant cells, prevention of metastasis, or the prevention of relapse in a patient who has been cured. Treatment of infection includes destruction of the infecting agent, inhibition of or interference with its growth or maturation, neutralization of its pathological effects, and the like.

The term "infection" as used herein includes infection by viruses, bacteria, fungi, and other parasites, such as leishmania and malarial parasites. "Infectious agents" within the scope of this invention include viruses such as HIV-I, HIV-II, HTLV-I, HTLV-II, herpes simplex virus (HSV), cytomegalovirus (CMV), Epstein-Barr virus (EBV), human papilloma viruses (HPV), hepatitis B virus (HBV), hepatitis C virus (HCV), polio virus, and the like; bacteria such as B. pertussis, and the causative agents of tetanus, diphtheria, cholera, and the like; mycobacteria such as M. tuberculosis, and the causative agent of leprosy; yeasts and fungi such as C. albicans and P. carinii; parasites such as malarial Plasmodia, giardia, and the like. Certain methods and constructs of the invention are most suitable for intracellular infectious agents, such as viruses, malaria and the like, while other methods and constructs are applicable to extracellular infections.

The term "hyperproliferative disorder" refers to disorders characterized by an abnormal or pathological proliferation of cells, for example, cancer, psoriasis, hyperplasia and the like.

The term "cis-acting regulatory sequence" refers to a polynucleotide sequence which is capable of responding to a trans-acting factor, and enhancing transcription of cis-located genes. Most appropriate cis-acting regulatory sequences may be derived from the infectious agent they will be used to combat. For example, the tar region of the HIV-1 LTR is a suitable cis-acting regulatory sequence for treatment of HIV-1. Where cell-type specific expression is desired, one may employ cis-acting sequences such as, for example, for T-cells, the CD2 antigen (Genbank HUMATCCD2), IL-2 (Genbank HUMIL2A), IL-2 receptor (Genbank HUMIL2R1), CD1 antigen (Genbank HUMHTA1), CD3 antigen (Genbank HUMATCT31), CD4 antigen (Genbank HUMATCT4), T-cell protease (Genbank MUSSPTCS); for B-cells, IgG (Genbank HUMIGCA1), MHC-1 antigen (Genbank HUMMHA2); for macrophages, Mac-1 antigen (Genbank HUMLAP), IL-1 Genbank (HUMIL1P), and the like. The cis-acting regulatory sequence controllable by a human disease-associated transacting regulatory factor is used in multiple tandem copies, in order to increase its competition with the endogenous cis-acting regulatory site. Suitable sequences for treatment of hyperproliferative disorders (especially cancers) are obtainable from the binding sites of known oncoproteins, or by identification of a known protein whose expression is altered by an oncogene. The cis-acting regulatory sequence must be capable of providing sufficient expression of the effector gene to confer susceptibility to protection or destruction of the cell when activated by the trans-acting factor, without allowing substantial constitutive expression in the absence of the trans-acting factor. The choice of cis-acting sequence will depend upon the trans-acting factor associated with the infection or disorder to be treated, and on the effector gene selected. For example, the HIV LTR contains both the tar region, which is highly selective for HIV tat, and also a region activated by the endogenous nuclear factor NF-_{κ}B (the LTR has tandem NF-_{κ}B binding regions). Although the tar sequence strongly suppresses expression in the absence of tat (see for example Muesing, Peterlin, supra), the cis-acting elements upstream of the tar sequence influence the degree of constitutive expression ("leakiness") that occurs in the absence of tat. The degree of leakiness in the absence of tat can be controlled by deletion or substitution of the cis-acting elements (e.g., NF-_{K}B binding sites) within the HIV-1 LTR.

The phrase "susceptible to protection or destruction" means that upon infection or occasion of a hyperproliferative disorder, the presence of the effector gene within the host cell renders the cell sensitive to an additional agent.

As indicated above, a polynucleotide construct of the invention expresses an enzyme or protein which renders the cell susceptible to an additional agent, for example, elevated expression of a nucleoside kinase may render the host cell susceptible to the action of gancyclovir or acyclovir, or may increase the potency of an anti-viral agent such as AZT. Dideoxynucleoside analogues (ddNs) such as AZT, dideoxycytidine, acyclovir, gancyclovir, and the like, are relatively non-toxic in their non-phosphorylated form. However, specific viral or intracellular enzymes may convert the ddNs to the corresponding triphosphates, at which point the agent acts as a chain termination agent during transcription or replication. The utility of ddNs is based on the fact that (1) viral polymerases exhibit a higher affinity for ddNs than mammalian polymerases, and (2) some viral nucleoside kinases exhibit a higher rate of phosphorylation for ddNs than mammalian kinases. Thus, viral enzymes (and consequently viral genes) are more affected by chain termination than are mammalian enzymes and genes. The HSV-1 thymidine kinase (HSV-1 tk) is more efficient at converting ddNs to the corresponding triphosphates, thus rendering cells having active HSV-1 tk more susceptible to ddNs.

Suitable effector genes for use in accordance with the invention include, in addition to genes encoding a nucleoside kinase capable of phosphorylating dideoxynucleoside analogues at a greater rate than host cell nucleoside kinases, for example HSV-1 thymidine kinase, guanine kinase, plant nucleoside phosphotransferase, Leishmania donovani purine 2'-deoxyribonucleosidease, L. donovani hypoxanthine-guanine phosphoribosyltransferase and other suitable enzymes of nucleoside metabolism capable of activating nucleoside antiviral or chemotherapeutic agents.

The effector gene may be under the control of a plurality of cis-acting sequences in conjunction with a strong termination sequence.

The term "vector" as used herein refers to any polynucleotide construct capable of encoding the cis-acting regulatory sequence and the effector gene(s) selected, and capable of transferring these genes into suitable target cells. Vectors may be linear or circular, double stranded or single stranded. Vectors may comprise DNA, RNA, DNA/RNA hybrids, and DNA and/or RNA polynucleotides having chemically modified bases. Suitable vectors within the scope of this invention include linear dsDNA segments, plasmids, recombinant viruses (e.g., recombinant vaccinia, adenovirus, adeno-associated viruses, and the like), replication-defective retroviral vectors (including human, simian, murine, and the like), suitably non-pathogenic derivatives of replication-competent retroviral vectors, and the like. Replication defective retroviral vectors are presently preferred.

### B. General Method

Preparation of polynucleotide constructs is conducted using methods known generally in the art. Once an infectious agent or hyperproliferative disorder has been selected for treatment, the first step is to identify an associated trans-acting factor, an appropriate cis-acting sequence, and an effector gene.

Some viral trans-acting factors are already known and characterized. Other viral trans-acting factors may be identified by deletion analysis of the cloned viral genome. For example, a new virus may be cloned and sequenced using standard techniques, and the open reading frames identified. Then, deletion mutants are prepared using restriction enzymes, and the mutant viruses assayed for transcriptional competence in suitable host cells. Mutants which exhibit very low mRNA transcription probably lack either a gene encoding a trans-acting factor, or a cis-acting regulatory sequence. Whether the deletion affects the trans-acting factor or the cis-acting sequence can generally be determined by examination of the position and content of the genomic sequence (e.g., cis-acting viral regulatory sequences are generally found upstream of open reading frames). The sequence of the cis-acting viral region may be compared for homology to known cis-acting regions. As homologous cis-acting sequences may react with the same trans-acting factor, appropriate endogenous trans-acting factors may thus be identified. Alternatively, viral proteins may be expressed recombinantly in appropriate hosts (e.g., bacteria, yeast, mammalian cell culture), and purified extracts labeled and screened for binding to the viral genome library. In this manner, appropriate trans-acting factor/cis-acting sequence pairs may be identified. The suitability of the pair may be evaluated using the CAT expression assay detailed below and in the examples. In these assays, the cis-acting sequence is cloned into a suitable vector, and a suitable reporter gene (e.g., CAT, β-galactosidase, etc.) ligated to the cis-acting sequence to provide for cis-control. The test construct is then transferred into a suitable host cell (e.g., mammalian cell culture) and assayed for reporter gene expression in the presence and absence of the trans-acting factor. Suitable effector genes are known in the art, or may be cloned using standard techniques.

Intracellular parasites (including viruses) often induce interferon expression. Thus, isolation of the interferon cis-acting sequence should allow identification of trans-acting factors involved in the anti-parasite response, and preparation of appropriate polynucleotide constructs of the invention.

Hyperproliferative disorders are characterized by inappropriate gene regulation or gene product activity, typically involving cell growth or differentiation factors, and occasionally genes encoding structural proteins. Inappropriate gene regulation may result from the expression of a dysfunctional trans-acting factor (e.g., a factor in which truncation or mutation has eliminated inhibition of the factor, or which binds irreversibly, etc.), from the overexpression of a trans-acting factor or receptor (e.g., due to the translocational juxtaposition of a strong promoter), or other defects. In any case, the disorder characteristically exhibits a trans-acting factor which is either different from those found in normal cells, or is present in abnormally large quantities. Trans-acting factors encoded by viruses associated with hyperproliferative disorders (e.g., HTLV-I, HTLV-II, the E6 and E7 genes of HPV type 16 and type 18) may also be used to regulate expression of an effector gene, as described herein. Once the affected cis-acting sequence is identified, a suitable effector gene may be selected. As the characteristic trans-acting factors are generally at least somewhat homologous to normal transacting factors, the cis-acting sequence is likely to exhibit some regulation by endogenous trans-acting factorsin normal cells. However, the activity of the trans-acting factors may be constitutively high in the hyperproliferative cells, whereas the activity may be lower and fluctuating (as during the cell cycle) in normal cells. This difference may be exploited to allow accumulation of high levels of effector gene product in hyperplastic cells.

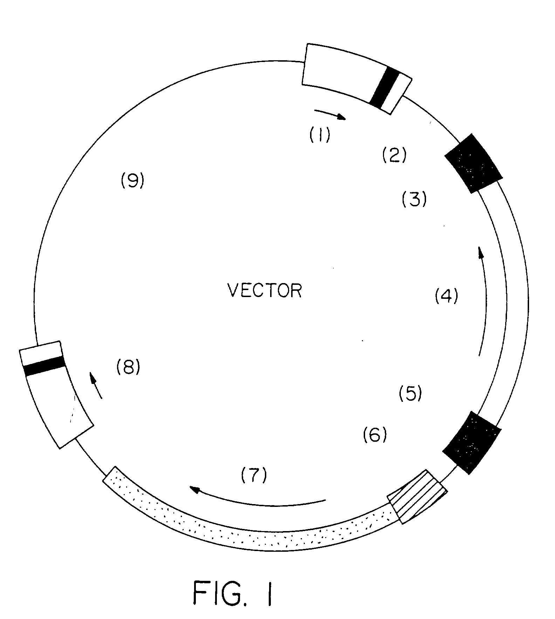

Referring to Figure 1, a retroviral vector of the invention is depicted apart from insertion of one or more further copies of the cis-acting regulatory sequence. The vector comprises a 5' retroviral LTR and primer binding site (1), a psi encapsidation (packaging) signal sequence (2), an optional 3' RNA processing signal sequence (3), an effector gene (4), a 5' cis-acting regulatory sequence (5) including a promoter, optionally a promoter (6) and selectable marker gene (7), and a 3' retroviral LTR and primer binding site (8). Sequences 1-8 constitute the retroviral portion of the vector, while sequence 9 is typically derived from a plasmid, and provides for maintenance of the vector in cell culture. The 5' LTR (1) and 3' LTR (8) provide for reverse transcription and integration of the vector into the host cell genome. Suitable LTRs include those derived from Moloney murine leukemia virus (Mo-MuLV) (Shinnick et al, Nature (1981) 293:543), Harvey murine sarcoma virus (Ha-MSV), (Van Beveran et al, Cell (1981) 27:97), HTLV-I, HTLV-II, HIV-1, and HIV-2. In replication-defective retroviruses, the U3 region of the 3' LTR (8) is inactivated. Psi sequence (2) must be included in order for the vector to be included in viral capsids generating in the packaging cell line, but is otherwise inactive once integrated into the host genome. Suitable psi sequences have been described by Cepko et al, Cell (1984) 37:1053-62; Guild et al, *supra;* and Kriegler et al, Cell (1984) 38:483. Where the vector is nonretro-viral, and is to be inserted by transfection rather than infection, the LTR sequences and psi sequence may be omitted. The effector gene (4) is as described above. In "internal promoter" recombinant retroviral vectors, the effector gene (4) is provided with its own promoter/cis-acting regulatory sequence (5) and terminator sequence/poly-adenylation sequence (4) (although the terminator and PA sequences may be derived from the effector gene).

The effector gene may be oriented in either direction, but is usually oriented in the direction opposite to the LTR reading frame. In "enhancer replacement" recombinant retroviral vectors, the effector gene (4) is oriented in the same direction as the LTR, and is not provided with a separate cis-acting regulatory sequence (5) or terminator sequence/polyadenylation sequence (4). Instead, the cis-acting regulatory sequence is provided within the 3' LTR (8) U3 region, so that the effector gene is controlled by the cis-acting regulatory region only after reverse transcription.

The selectable marker (7), if present, encodes a characteristic which enables cells expressing the marker sequence to survive under conditions selecting for transfected cells. The selectable marker typically encodes an enzyme conferring resistance to an antibiotic, for example chloramphenicol, neomycin (Southern et al, J Mol Appl Gen (1982) 1:327-41), or hygromycin (Gritz et al, Gene (1983) 25:179-88). The selectable marker, when employed, is usually provided with its own promoter (6) which allows expression of the marker gene during selection of transfected/infected cells. Suitable marker gene promoters include the histone promoter, HSV-1 tk promoter, and the metallothionein promoter.

Sequence 9 is a polynucleotide maintenance sequence, which provides for the stable maintenance of the vector within producer cells. Typically, sequence 9 is derived from a plasmid, and provides an origin of replication (such as pBR322 ori, or the yeast 2µ origin) and (usually) an antibiotic resistance marker. Suitable maintenance sequences include pXf3, pBR322, pUC18, pML, and the like. In the case of linear DNA segments used for targeted integration, the vector may be linearized at a point within sequence 9. The LTR regions 1 and 8 are replaced with sequences homologous to the sequence of desired targeted recombination. Sequence 2 is deleted. Sequence 9 includes polynucleotide sequences which provide for maintenance and replication in microbial hosts, and additionally includes a counter-selection marker (which provides for deletion of transfected host cells which undergo non-specific integration).

### Development of drug activation systems

As a prototype system for activation of cytotoxic drugs, we chose the thymidine kinase gene from Herpes Simplex Virus type 1 (HSV-1 tk). HSV-1 tk can activate a variety of dideoxynucleoside analogues (ddNs) by conversion of these drugs to the (5') monophosphate species. The ddNMPs can act as competitive inhibitors of cellular nucleotide or nucleoside kinases, causing depletion of cellular nucleotide pools; following conversion to triphosphate species (ddNTPs) by cellular enzymes, ddNTPs can be incorporated into nascent DNA (and possibly RNA) strands, causing chain termination.

One of the best-characterized ddNs which can be activated by HSV-1 tk is acyclovir (acyclo-G). The safety and efficacy of acyclovir derives primarily from its selective activation by HSV-1 tk: acyclo-G is converted to acyclo-GMP several thousandfold more efficiently by HSV-1 thymidine kinase (tk) than by cellular thymidine kinases (Km for HSV-1 tk = 0.005X Km Vero tk; Vrel HSV-1 tk = 3,000,000X Vrel Vero tk). Cellular enzymes then convert acyclo-GMP to acyclo-GTP, which is incorporated into DNA by HSV-1 DNA polymerase causing termination of viral DNA synthesis. Acyclo-GTP inhibits HSV-1 DNA synthesis at about 1/20 the concentration required for similar inhibition of cellular DNA synthesis (P.A. Furman et al, J Virol (1979) 32:72-77).

Once a cis-acting regulatory sequence has been identified, it is tested for effect in a model system. For example, the cis-acting regulatory sequence may be cloned into a plasmid with a suitable reporter gene, such as CAT or β-galactosidase. The plasmid is then transfected into a suitable cell line (e.g., CHO cells, HeLa, HUT78, and the like). The trans-acting factor may be supplied by selecting a host cell line which expresses the trans-acting factor, or by cotransfecting the host cell line with a plasmid encoding the trans-acting factor under control of a different promoter (either inducible or constitutive). The transfected host cells are then assayed for expression of the reporter gene.

The suitability of an effector gene such as HSV-1 tk is assessed by cloning into a suitable plasmid under control of the selected cis-acting regulatory region. The plasmid also preferably contains a selectable marker, allowing one to select those cells which have become genetically transformed by the vector.

### General structure of thymidine kinase vectors

The general structure of retroviral vectors which can be used to generate recombinant retroviruses containing an effector gene linked to cis-acting regulatory elements is shown in Figure 1. The basic retroviral vectors for expression of HSV-1 tk are derived from Moloney Murine Leukemia Virus (Mo-MuLV). All of the Mo-MuLV genes (gag, pol and env) have been removed from the vectors in order to generate completely replication-defective viruses. The remaining components are required for expression and packaging of viral RNA, reverse transcription and integration, and transcription of the tk gene (and marker gene, if desired) in the target cells. These components consist of the Mo-MuLV Long Terminal Repeats (LTRs), the plus- and minus-strand primer binding sites, and the RNA encapsidation signal (Psi sequence).

In order to regulate the expression of HSV-1 tk in a cell type-specific manner, a hybrid transcription unit is constructed in which the HSV-1 tk coding sequence replaces the coding sequence of a cell type-specific transcription unit.

Expression of the HSV-1 tk gene via these vectors can be accomplished in two ways. The first type of vector uses a separate enhancer/regulatory element to regulate expression of HSV-1 tk. In this type of vector, transcription of HSV-1 tk proceeds in the opposite orientation to the plus-sense of the provirus (as shown in Figure 1). The tk gene is provided with its own polyadenylation signal, and, if desired, also can be provided with an intron between the tk coding sequence and polyadenylation signal. Potential transcriptional interference in this first class of vectors can be reduced by removing the enhancer/regulatory element sequences from the 3' LTR of the vector. Because only the U3 sequences of the 3' LTR are transcribed into viral RNA, these sequences form both LTRs in the resultant provirus. This allows integration of the proviral cDNA, but results in loss of the enhancers and regulatory elements from both LTRs of the provirus.

In the second type of vector (enhancer replacement vector), the Mo-MLV LTR provides the enhancer and regulatory element sequences that control expression of the HSV-1 tk gene. The Mo-MLV LTR can drive expression of heterologous genes in a variety of cell types (excluding hematopoietic stem cells), but is most efficient in T-cells. Control of cell type-specific gene expression of HSV-1 tk may be achieved by substitution of specific enhancer/regulatory element sequences for the enhancer/regulatory element sequences within the 3' Mo-MLV LTR. The 5' Mo-MLV LTR is retained in the vector to allow efficient expression of viral RNA in the packaging cell line used to generate infectious virus.

A selectable marker gene can be incorporated into these vectors 3' to the HSV-1 tk gene in order to allow in vitro selection of transformed cells. Expression of the marker gene is provided by its own regulatory element. In general, this regulatory element would be derived from a moderately active cellular gene that is constitutively expressed in all tissues. Some examples of such regulatory elements would be the cytoplasmic B actin promoter, histone promoters, and promoters for glycolytic enzymes.

The second type of vector uses a separate enhancer/regulatory element to regulate expression of HSV-1 tk. In this type of vector, transcription of HSV-1 tk proceeds in the opposite orientation to the plus-sense of the provirus. The tk gene is provided with its own polyadenylation signal, and, if desired, also can be provided with an intron between the tk coding sequence and polyadenylation signal. Potential transcriptional interference in this second class of vectors can be reduced by removing the enhancer/regulatory element sequences from the 3' LTR of the vector. This allows integration of the proviral cDNA, but results in loss of the enhancers and regulatory elements from both LTRs of the provirus.

A selectable marker can be provided, as in the first class of vectors, and is transcribed in the opposite direction from HSV-1 tk. Transcription thus initiates in the center of the integrated provirus and proceeds in opposite directions, in a manner analogous to the transcription of the E2/E3 genes of human adenoviruses.

### Formulation and Administration

The polynucleotide constructs of the invention may be formulated, depending on the form of construct. For example, where the vector is a competent (infectious) virus, it may be formulated in the same manner as live virus vaccines. Such formulations are typically buffered, physiologic saline for injection, or buffered oral formulations, either of which may optionally contain antibiotics. Liposome formulations may be prepared by standard methods, for example by suspending lipids in chloroform, drying the lipids onto the walls of a vessel, and hydrating the lipids with a solution containing the polynucleotide construct. Suitable lipids are known in the art, including phosphatidyl serine, phosphatidyl glycerol, lethicin, and the like. A synthetic lipid particularly useful for polynucleotide transfection is N-[1-(2,3-dioleyloxy)propyl]-N,N,N-trimethylammonium chloride, which is commercially available under the name Lipofectin® (available from BRL, Gaithersburg, MD), and is described by P.L. Felgner et al, Proc Nat Acad Sci USA (1987) 84:7413.

Recombinant retroviral vectors may also be formulated for *in vivo* administration (if replication defective). Vectors which are based on HIV-1 or HIV-2 will demonstrate the same cellular tropisms as the native virus, thus providing an efficient and comprehensive means for targeting the appropriate cell populations *in vivo* when treating HIV-1 or HIV-2 infection. Vectors based on HIV may employ the HIV LTR promoters, tar sequence, and the tat gene for control of expression of heterologous genes. One may refine the target cell specificity of the vector by an appropriate choice of packaging constructs (e.g., env gene). For example, the isolate HIV-1_{SF162} (M. Quiroga et al, "Modern Approaches to New Vaccines" (1989, Cold Spring Harbor Laboratories) p. 80) is naturally selective for macrophage infection: thus, by packaging the vector in an HIV-1_{SF162}-derived envelope, targeting to the macrophages can be effected. Similarly, packaging the vectors using recombinant envelope genes which incorporate sequences derived from envelopes or capsids derived of hepatitis B virus (or HAV, or HCV) to obtain targeted delivery to hepatocytes. By using a packaging cell line expressing envelope proteins derived from HIV-2, one can obtain an effective method for targeting the vectors of the invention for protection of T_{H}-cells, due to the affinity of gp160^{env} for CD4⁺ (Smith et al, Science (1987) 238:1704-06).

The mode of administration of polynucleotide constructs of the invention depends on the nature of the vector. For example, retroviral vectors may be administered by inoculation, parenterally or orally. Where the vector is an infectious recombinant virus, administration may be by parenteral injection, oral or intranasal administration, and the like. Liposome formulations are preferably administered by intranasal spray or intravenous injection. The presently preferred method of administration is by incubating defective retroviral vectors with aspirated autologous bone marrow cells or T lymphocytes *ex vivo*, followed by reintroduction of the treated cells by standard techniques. Briefly, bone marrow cells are aspirated by techniques known in the art for bone marrow transplants, and are generally aspirated from the pelvis. If desired (particularly in the treatment of leukemia and other hyperplastic disorders), the subject may be treated with chemotherapy or radiotherapy following bone marrow aspiration, in order to reduce the burden of infected or hyperproliferating cells. The aspirate may be screened to remove undesirable material (e.g., tumor cells, bacteria, viral particles, and the like), for example by immunoprecipitation, immunoadsorption, immunoreaction with complement fixation, fluorescence-activated cell sorting (FACS), and the like. Ideally, hematopoietic stem cells are labeled and isolated using stem cell-specific MAbs. The screened aspirate is then transfected or infected with a construct of the invention. One method of infection is to maintain the aspirated cells in culture in contact with infectious titers of replication-defective retroviral vectors for a period sufficient to insure efficient inoculation. Growth factors for hematopoietic cells (for example IL-3) may be added during cocultivation (E.A. Dzierzak, *supra*). The calcium phosphate transfection techniques known for murine cells may be used (see for example, E.A. Dzierzak, *supra;* S-F. Yu et al, Proc Nat Acad Sci USA (1986) 83:3194-98). Constructs may also be introduced by electroporation (S. Mansour, *supra*). Alternatively, one may employ a transfection agent such as Lipofectin®. Where the vector includes a marker, the infected cells may be screened or selected, if desired. The infected cells are then reintroduced into the subject using methods employed for autologous bone marrow transplantation, typically by intravenous infusion or injection. One may also transfect or infect lymphocytes either *in vivo* or *ex vivo* using constructs of the invention. Infection using MLV-based constructs is preferably conducted *ex vivo*, as the envelope is inactivated by human complement. Specific subsets of the lymphocyte population may be selected by employing monoclonal antibodies to separate the desired subset, or by other methods known in the art: see for example P.W. Kantoff et al, Proc Nat Acad Sci USA (1986) 83:6563-67.

If desired, one may convert some of the lymphocytes or bone marrow cells to packaging cells, by inserting independent constructs capable of expressing the viral genes encoding gag-pol and env, followed by insertion of a vector of the invention. O. Danos et al, Proc Nat Acad Sci USA (1988) 85:6460-64. When the autologous packaging cells are reintroduced into the subject, continuous expression of replication-defective retrovirus during the cell lifetime results in transfer of the therapeutic construct of the invention to a large number of host cells.

### C. Examples

The examples presented below are provided as a further guide to the practitioner of ordinary skill in the art, and are not to be construed as limiting the invention in any way.

### EXAMPLE 1

### Recombinant Retroviral Vectors

Retroviral vectors were constructed using standard techniques for manipulation of recombinant DNA (T. Maniatis et al., Molecular Cloning: A Laboratory Manual (New York, Cold Spring Harbor Laboratory, 1982)). Plasmids used in construction of retroviral vectors were obtained from the following sources: pMX1112SVNeo was a gift from M. McMann (UCSF), SV-N was a gift from E. Gilboa (constructed as described by Yu, *supra*).

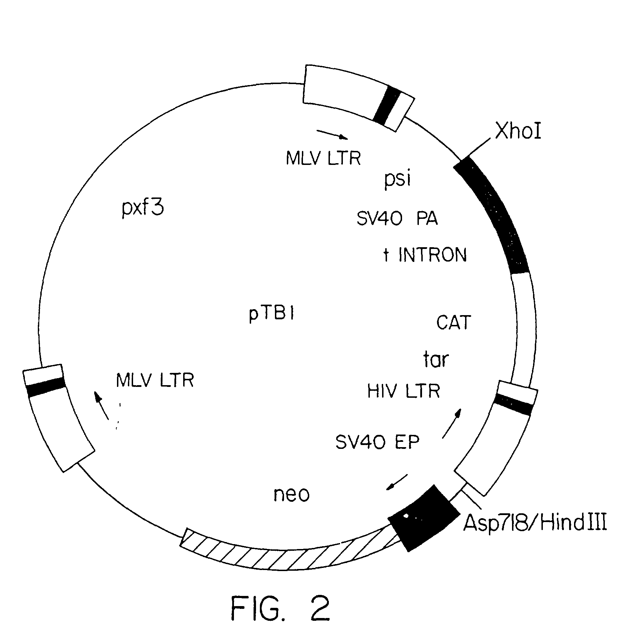

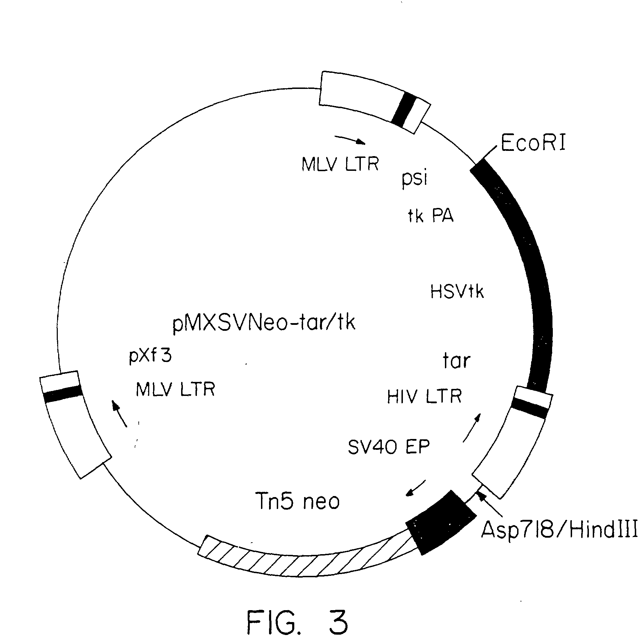

Plasmid pMX1112SVNeo is constructed by inserting a Cla-Cla fragment of the SV40 early promoter linked to the neo^{r} gene into the plasmid pMX1112 (described by A.M.C. Brown et al, "Retroviral Vectors", pp. 189-212, in "DNA Cloning: a practical approach, vol. 3 (D.M. Glover, ed, IRL Press, 1987)). The SV40-neo^{r} gene is positioned between the psi packaging sequence and the 3' Mo-MuLV LTR region on the plasmid. Plasmid pMXSVNeo18 was prepared from pHX1112SVNeo by removing the EcoRI site upstream of the 5' Mo-MuLV LTR, inserting an EcoRI linker at the XhoI site, and adding the pUC18 polylinker between the new EcoRI site and the HindIII site. Plasmid pTAR1 contains the HIV 5' LTR (including the tar sequence), a gene encoding CAT, and an SV40 polyadenylation signal and t intron, and was constructed by cloning the XhoI-NarI fragment of HIV-1 (SF2) (R. Sanchez-Pescador et al, Science (1982) 227:484-92) into pML. A synthetic polylinker was added at the NarI site, and the Stul-BamHI fragment of pSV2CAT (C.M. Gorman et al, Proc Nat Acad Sci USA (1982) 79:6777-81) containing the CAT gene and SV40 RNA processing signals was inserted to provide pTAR1. The pTAR1 was cleaved with Asp718 and XhoI, and the resulting fragment cloned into pMXSVNeo18 to produce pTB1, shown in Figure 2. Plasmid pTAT1 was constructed with the HIV tat gene under control of the SV40 EP, and flanked by the SV40 polyadenylation signal (Peterlin et al, *supra).* Plasmid pRT contains the HSV-1 tk gene, promoter, and RNA processing signals. The plasmid ptar/tk was prepared by cloning the BglII-EcoRI fragment of pRT (containing the tk coding sequence and polyadenylation signal) into pTAR1 in place of the CAT gene. Plasmid pMXSVNeo-tar/tk was constructed by cloning the Asp718-EcoRI portion of ptar/tk into the pMXSVNeo18 polylinker (pMXSVNeo-tar/tk is depicted in Figure 3).

Amphotropic retroviruses capable of infecting human cells were prepared by standard techniques (R. Mann et al, Cell (1983) 33:153-59). Briefly, the amphotropic packaging cell line PA-317 (Miller et al, Mol Cell Biol (1986) 6:2895-902; ATCC CRL 9078) was transfected with plasmid vector using the CaPO₄ coprecipitation technique (R. Graham et al, Virol (1973) 52:456-67). 10 µg of plasmid vector plus 25 µg salmon sperm DNA carrier were applied as a CaPO₄ coprecipitate to 5 x 10⁵ PA-317 cells in a 60 mm tissue culture dish for 8-16 h. The precipitate was removed and the monolayer was rinsed with Dulbecco's phosphate-buffered saline. Fresh medium (Dulbecco's modified Eagles medium, DMEM, supplemented with 10% fetal bovine serum and antibiotics) was applied and the cells were allowed to grow and recover for 2 d. The transfected cells were then trypsinized and transferred to a 150 mm2 T-flask and grown for 10-14 d in medium containing 0.8 mg/mL G418. The resulting G418-resistant colonies were trypsinized and cloned by limiting dilution in 96-well tissue culture plates. Recombinant retrovirus produced by individual clones was characterized by infection of human cell lines (HeLa, ATCC CCL 2: HUT78, ATCC TIB 161) to determine viral titer and generation of the correct proviral structure.

Ecotropic recombinant retroviruses were prepared as described above, except that the ecotropic packaging cell line Psi-2 (R. Mann, *supra*) was used instead of PA-317.

### EXAMPLE 2

### Infection of cells with recombinant retroviruses

Cell lines were infected with recombinant retroviruses using standard procedures (R. Mann, *supra*). Briefly, 5 x 10⁶ cells were plated into 60 mm tissue culture dishes and allowed to grow for 16 h. Cell-free supernatants from packaging cells containing plasmid vectors was applied to the target cells for 6-8 h in the presence of 8 µg/mL polybrene. In the case of viruses carrying the G418-resistance marker, cells were grown, selected, and cloned as described for preparation of packing clones, above. Retroviruses carrying HSV-1 tk, but lacking the G418-resistance marker were titered using tk- cell lines: L-M (tk-) (ATCC CCL 1.3) for ecotropic viruses, 143 B cells (ATCC CRL 8303) for amphotropic viruses. Selection for tk⁺ colonies was performed using HAT medium.

### EXAMPLE 3

### Assay For Trans-activation by HIV tat

Plasmids pTAR1, pTB1, and pTB2 (having the HIV LTR region in the opposite orientation from pTB1), encoding CAT under control of the HIV tar cis-acting regulatory sequence, were transfected into HeLa cells. Plasmid pTAT1, expressing the HIV tat trans-acting agent, was transfected into half of the HeLa cells. After 48 hours, the cells were lysed, and ¹⁴C-chloramphenicol was incubated with the lysates. The products were extracted with EtOAc, and analyzed by thin-layer chromatography.

The results indicated that CAT was expressed in HeLa cells which contained both a tar-encoding plasmid and a tat-encoding plasmid, but not in HeLa cells lacking the pTAT1 plasmid. Although the level of constitutive expression (leakiness) varied little between pTAR1, pTB1, and pTB2, CAT expression in response to HIV tat was higher in cells containing pTB1 and pTB2 than in cells containing pTAR1 (lacking the Mo-MuLV LTRs and SV40 enhancer).

### EXAMPLE 4

### Antiviral Cytotoxic Effector Genes

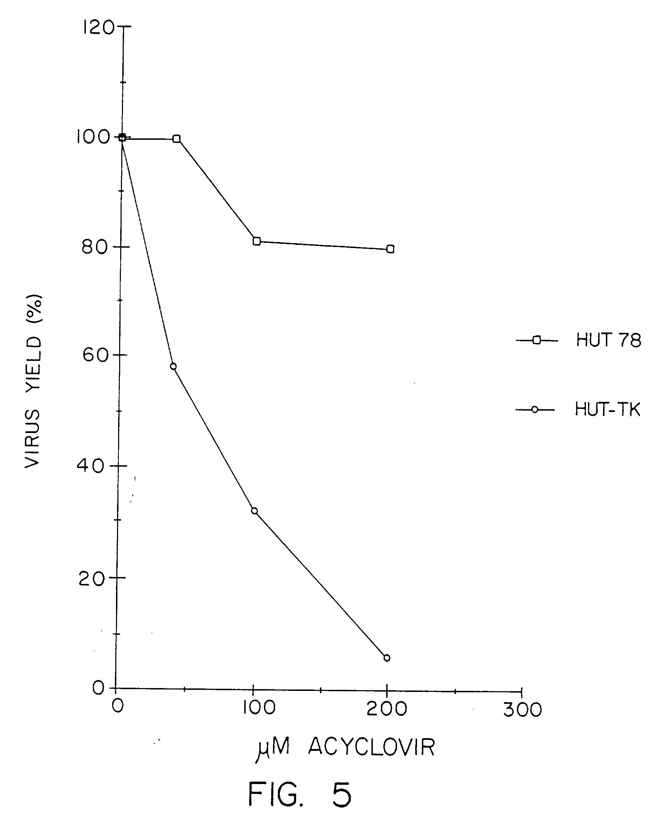

Amphotropic MXSVNeo-tar/tk retrovirus was produced, and used to infect HUT78 cells (ATCC TIB 161). Transformed cells ("HUT-tk cells") were selected using 1 mg/mL G418. The sensitivity of mass-cultured HUT-tk cells to acyclovir was determined by growth in medium containing various concentrations of drug. HUT78 cells containing pMXSVNeo-tar/tk ("HUT-tk cells") were pelleted and exposed to polybrene (2 µg/mL) at 37°C for 30 minutes. Cells were then pelleted and resuspended at 10⁵ cells per mL, and exposed to HIV-1 (SF2) for 1.5 hours at 37°C. The cells were then pelleted and resuspended in RPMI 1640 medium containing 10% fetal calf serum and antibiotics at 104 cells per 50 µL. Fifty µL of cell suspension was then distributed to wells of a 24 well plate containing 1 mL of medium with 45, 100, or 200 µM acyclovir. HIV-SF2 was added (sufficient 5 to provide 3 OD in a p25 gag ELISA after 7 days of incubation), and was allowed to propagate for 7 days, and then assayed by anti-p25gag ELISA. Normal HUT78 cells served as controls for the effect of HSV-1 tk. The results of these experiments are shown in Figure 5.

Although HIV replication in HUT78 cells was not significantly affected by acyclovir, replication was strongly inhibited by acyclovir in HUT-tk cells (about 60% with 100 µM acyclovir). Cytopathic/cytotoxic effects were apparent in both cell types (data not shown). Although 5 analysis of single cell clones of HUT-tk showed variation in sensitivity to acyclovir, the mass-cultured HUT-tk cells were capable of significantly restricting viral replication.

### EXAMPLE 5

### Assay for inhibition of HIV replication

HIV-infectable cells transformed to an HSV-1 tk positive phenotype can be used for screening nucleoside analogues for antiviral activity and cellular cytotoxicity (Mitsuya, *supra*).

HUT78 cells containing pMXSVNeo-tar/tk ("HUT-tk cells") were cloned by limiting dilution and assayed for sensitivity to varying concentrations of acyclovir as a measure of constitutive tk expression. An especially sensitive clone was designated TK.5 and was chosen to test the ability of cells expressing high levels of tk to activate AZT and resist HIV infection.

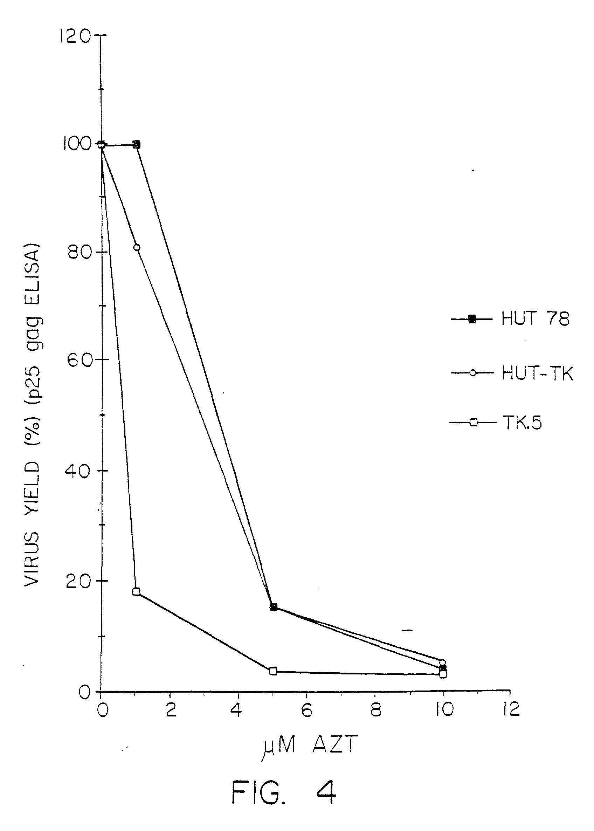

HUT78, mass-cultured HUT-tk (from Example 4), and TK.5 cells were pelleted and exposed to polybrene (2 µg/mL) at 37°C for 30 minutes. Cells were then pelleted and resuspended at 10⁵ cells per mL, and exposed to HIV-1 (SF2) for 1.5 hours at 37°C. The cells were then pelleted and resuspended in RPMI 1640 medium containing serum and antibiotics at 10⁵ cells per 50 µL. Fifty µL of cell suspension was then distributed to individual wells of a 24 well plate containing 1 mL of medium with 0, 1, 5, or 10 µM AZT. The infected cells were culture for 7 days, and virus replication was then assayed by anti-p25gag ELISA. The results are set forth in Figure 4.

### EXAMPLE 6

### Construction of specific thymidine kinase vectors

The structure of various retroviral vectors containing HSV-1 tk is shown in Figure 1. The functional characteristics and therapeutic applications of these vectors are described below.

SIN-tar/tk-H4Neo: This vector employs the self-inactivating vector described by Yu, using the HIV tar cis-acting sequence and the HSV-1 tk effector gene, and containing neomycin resistance under control of the H4 histone promoter. This plasmid confers susceptibility to ddNs on cells infected with HIV.

SIN-CD4/tk (-H4Neo): This vector is identical to the SIN-tar/tk-H4Neo vector described above, except that the tar cis-acting sequence is replaced with the cis-acting sequence which promotes expression of CD4 antigen in T_{H} cells. The histone promoter/neomycin resistance marker is optional. This vector confers susceptibility to ddNs on all CD4⁺ cells. As the CD4 antigen is currently believed to be important in the mode of entry for HIV, this vector would also be useful in the treatment of HIV infection.

SIN-Mac1/tk (-H4Neo): This vector is like SIN-tar/tk-H4Neo, with the HIV tar cis-acting sequence replaced by the cis-acting sequence regulating Mac1 antigen expression in macrophages. The histone promoter/neomycin resistance marker is optional. This vector confers susceptibility to ddNs on all macrophages, which also serve as host cells to HIV.

SIN-tar/rA-H4Neo: This vector employs the HIV-1 tar cis-acting sequence, controlling the ricin A effector gene, using neo^{r} as a selectable marker.

SIN-fos/ppt: This vector employs the self-inactivating vector described by Yu, using the fos oncogene cis-acting sequence (R. Treisman Cell (1986) 46:567-74) using a plant phosphotransferase effector gene. This vector, in combination with a ddN, would be useful in the treatment of fos-type malignancies.

SIN-pcna/tnf: This vector uses the cis-acting sequence regulating Proliferating Cell Nuclear Antigen (described by J.E. Selis, Leukemia (1988) 2:561-601) and a tumor necrosis factor effector gene. Although PCNA is expressed in all cells, the expression is transient, and occurs only during cell division. Thus, normal cells infected with the vector would not produce toxic concentrations of TNF, whereas neoplastic hyperproliferating cells which are constantly in cell division would express toxic concentrations.

SIN-acg/ifn: This vector employs the cis-acting regulatory sequence which provides expression of α chorionic gonadotropin (S.E. Goelz, Science (1985) 228:187-90) in combination with a β-interferon effector gene. This vector, like the preceding vector, relies on the fact that acg is infrequently expressed in normal cells, and would be useful in cancer treatment.

SIN-IgG/Rbz: This vector employs the cis-acting sequence from IgG, along with a ribozyme effector gene specific for the bcl/abl splice site which occurs in chronic myelogenous leukemia (CML). As IgG is expressed in B-cells, cell-type protection against CML is obtained.

SIN-e7/Ld: This vector uses the cis-acting sequence responding to the E7 gene of human papilloma virus type 16 (HPV16) in conjunction with a Leishmania donovani purine 2'-deoxyribonucleosidease gene. As expression of HPV16 E7 gene is associated with carcinoma of the uterine cervix (Phelps et al, Cell (1988) 53:539-47), this vector (in combination with the drug 6-methylpurine 2-deoxyriboside) destroys cells in which the E7 gene is expressed.

HIV-2/tk: This vector is derived from HIV-2 from which all of the internal genes are deleted except for the portion of the gag gene which overlaps the psi (encapsidation) signal and the rev-response element from the env gene. The HSV-1 tk gene is inserted 3' from the psi sequence, and is expressed under control of the HIV-2 LTR. If desired, the HIV-1 tar sequence can be substituted for the HIV-2 tar sequence. This vector may be used to express HSV-1 tk from a genome-length RNA by deleting or altering the HIV gag sequence ATG codons (Guild et al, *supra*), obtaining expression of the HSV-1 tk gene from its own ATG start codon. Alternatively, one may include the HIV-2 env splice acceptor just 5' of the HSV-1 tk gene, without altering the gag start codons, thus obtaining expression of the HSV-1 tk via a spliced subgenomic mRNA. In either case, expression of HSV-1 tk is dependent upon complementation by tat from either a resident HIV-1 provirus or a superinfecting HIV-1 virus. Expression of tk in combination with administration of ACV or GCV then activates toxic levels of the antiviral agent, thus destroying the infected cell. These constructs may be packaged in either HIV-2 envelopes or in HIV-1_{*SF162*} envelopes, the latter being useful for targeting and destroying infected macrophages.

HIV-2/TCR-tk: This construct incorporates the T-cell receptor promoter internally to drive expression of HSV-1 tk, and contains the RRE from env and psi signal from the 5' end of the HIV-2 gag gene. The 3' LTR of the vector is modified by inserting the TCR α constant region enhancer sequence (described by Ho et al, Proc Nat Acad Sci USA (1989) 86:6714), according the principle disclosed by J. Overhauser et al, J Virol (1985) 54:133-34 for construction of a glucocorticoid-responsive MoMLV. The HIV-2/TCR-tk vector may be used to infect and genetically transform CD4⁺ cells *in vivo.* As neither the vector nor the T4 cell normally contains tat, no expression of transcripts initiating in the viral LTR occurs in the absence of infection with HIV. Initiation of transcription (expression of tk) occurs from the internal TCR promoter. Expression from this promoter is enhanced by the presence of the TCR α enhancers incorporated into the proviral LTRs. This vector provides constitutive expression of tk in the genetically transformed T_{H} lymphocytes, which in turn activates an antiviral agent (e.g., ACV, ddC and the like). This permits the use of ACV and related compounds in cells in which they would normally not be effective. It also increases the effectivness of AZT at low concentrations as shown in Figure 4. The ability to employ a variety of antiviral agents should reduce the possibility of development of drug resistance by HIV. An analogous vector may be prepared by substituting MLV virus components for the HIV sequences used, and employing an MLV amphotropic packaging cell line.

### EXAMPLE 7

### In Vivo Administration of Constructs

(A) Packaging cell lines for HIV-based vectors are constructed as follows:

The gag-pol coding sequence is isolated from HIV-1 or HIV-2, and is inserted into an expression vector under control of the human cytomegalovirus (CMV) major immediate early (MIE) promoter. A suitable polyadenylation sequence is provided 3' from the gag-pol insertion, for example, the SV40 polyA coding sequence. Both the gag-pol and the env sequences must contain the rev-response element (RRE) present in the env coding region, in order to obtain nuclear export (M.H. Malim et al, Nature (1989) 338:254-57).

The env sequence (including the RRE), which will determine the ultimate specificity of the finished vector, is obtained from a selected HIV-1, HIV-2 or SIV-1 isolate, and is inserted into a separate expression vector under control of the CMV MIE promoter. In this construction, a β-globin polyA sequence is employed. An intron may be inserted 5' to the env coding sequence, optionally containing a coding sequence for a marker or reporter gene (e.g., neo^{r}).

Separate similar vectors are constructed to insert tat cDNA and rev cDNA, under control of the SV40 early promoter. These vectors employ SV40 polyA sequences. By separating the coding sequences for gag-pol, env, tat, and rev, the possibility of recombination to regenerate a functional HIV virus is minimized.

The expression vectors are then used to construct a packaging cell line, by transfection of suitable cell line, for example NIH-3T3, HeLa, or the like. Upon transfection with a retroviral vector of the invention such as HIV-2/TCR-tk, a protective construct of the invention is prepared which is capable of infecting the population of host cells that is infected during *bona fide* HIV infection (mainly T_{H} cells). When administered in combination with suitable antiviral agents such as AZT, ACV, ddC or the like, this treatment protects infected cells from infectious HIV replication.

(B) Macrophage/monocyte-trophic constructs are prepared following the procedure of part A above, but employing the HIV-1_{SF162} env gene to provide env protein. Upon transfection with a vector of the invention such as HIV-2/tk, an ablative construct is provided which is capable of superinfecting and destroying host cells which are reservoirs of HIV and infection, particularly macrophages and monocytes.

## Claims

1. A polynucleotide construct for treating a host cell for a hyperproliferative disorder or infection by an infectious agent, said infection or hyperproliferative disorder being **characterised by** a human disease-associated transacting regulatory factor capable of regulating expression of genes, which construct comprises:
(i) at least two tandem copies of a cis-acting regulatory sequence which are controllable by said trans-acting regulatory factor; and
(ii) an effector gene under the control of said cis-acting regulatory sequence, said effector gene encoding an enzyme which renders said cell susceptible to an additional agent.

2. A polynucleotide construct as claimed in claim 1 wherein said effector gene encodes a nucleoside kinase capable of phosphorylating dideoxynucleoside analogues at a greater rate than host cell nucleoside kinases.

3. A polynucleotide construct as claimed in claim 2 wherein said effector gene encodes herpes simplex virus type 1 thymidine kinase (HSV-1 tk)

4. A polynucleotide construct as claimed in any one of claims 1 to 3 wherein said cis-acting regulatory sequence is capable of responding to HIV tat protein.

5. A polynucleotide construct as claimed in any one of claims 1 to 3 wherein said cis-acting regulatory sequence is capable of directing cell-specific expression.

6. A polynucleotide construct as claimed in any one of claims 1 to 5 which further comprises a polynucleotide maintenance sequence which provides for the stable maintenance of said construct within mammalian cells.

7. A polynucleotide construct as claimed in claim 6 which is in the form of a viral vector or a gene target vector.

8. A polynucleotide construct as claimed in claim 7 which is a recombinant viral vector selected from vaccinia, HIV-1, HIV-2, adenovirus and adeno-associated virus.

9. A polynucleotide construct as claimed in claim 7 which is a replication-defective retroviral vector.

10. A polynucleotide construct as claimed in claim 9 which further comprises a gp160^{env} glycoprotein coding sequence derived from HIV-1 or HIV-2.

11. A viral particle containing a viral vector as claimed in any one of claims 7 to 10.

12. A viral particle according to claim 11 which is an infectious replication-defective recombinant retrovirus.

13. A composition for treating a host cell for a hyperproliferative disorder or infection by an infectious agent which composition comprises a viral particle according to claim 11 or claim 12 and a pharmaceutically acceptable carrier.

14. A composition for treating a host cell for a hyperproliferative disorder or infection by an infectious agent which composition comprises a polynucleotide construct as claimed in any one of claims 1 to 10 and a pharmaceutically acceptable carrier.

15. A composition as claimed in claim 14 wherein said pharmaceutically acceptable carrier comprises a liposome.

16. A composition as claimed in claim 15 wherein said liposome further comprises antibodies specific for host cells desired to be treated

17. A construct as claimed in any one of claims 1 to 10 or a viral particle as claimed in claim 11 or claim 12 for use in a method of therapeutic treatment.

18. A method for treating a host cell *ex vivo* for a hyperproliferative disorder or infection by an infectious agent, said infection or hyperproliferative disorder being **characterised by** a human disease-associated trans-acting regulatory factor capable of regulating expression of genes, which method comprises inserting into said cell a polynucleotide construct as claimed in any one of claims 1 to 10.

19. A method as claimed in claim 18 wherein said *ex vivo* host cell is infected with a viral particle according to claim 11 or claim 12.

20. *Ex vivo* cells containing a polynucleotide construct as claimed in any one of claims 1 to 10.

21. Cells as claimed in claim 20 for use in a method of therapeutic treatment.

## Patentansprüche

1. Polynucleotid-Konstrukt zur Behandlung einer hyperproliferativen Krankheit oder Infektion durch ein infektiöses Agens bei einer Wirtszelle, wobei die Infektion oder hyperproliferative Krankheit durch einen mit einer humanen Krankheit assoziierten trans-wirkenden Regulationsfaktor **gekennzeichnet** ist, der fähig ist, die Expression von Genen zu regulieren, wobei das Konstrukt umfasst:
(i) mindestens zwei Tandemkopien einer cis-wirkenden Regulationssequenz, die durch den trans-wirkenden Faktor kontrollierbar sind; und
(ii) ein Effektorgen unter der Kontrolle der cis-wirkenden Regulationssequenz, wobei das Effektorgen für ein Ensym codiert, das die Zelle für ein zusätzliches Agens empfänglich macht.

2. Polynucleotid-Konstrukt nach Anspruch 1, wobei das Effektorgen für eine Nucleosidkinase codiert, die fähig ist, Didesoxynucleotid-Analoge mit einer größeren Rate zu phosphorylieren als Wirtszellen-Nucleosidkinasen.

3. Polynucleotid-Konstrukt nach Anspruch 2, wobei das Effektorgen für Herpes simplex-Virus Typ 1-Thymidinkinase (HSV-1 tk) codiert.

4. Polynucleotid-Konstrukt nach einem der Ansprüche 1 bis 3, wobei die cis-wirkende Regulationssequenz fähig ist, auf HIVtat-Protein zu reagieren.

5. Polynucleotid-Konstrukt nach einem der Ansprüche 1 bis 3, wobei die cis-wirkende Regulationsseguenz fähig ist, eine zellspezifische Expression zu steuern.

6. Polynucleotid-Konstrukt nach einem der Ansprüche 1 bis 5, das außerdem eine Polynucleotid-Aufrechterhaltungs-Sequenz umfasst, die für die stabile Aufrechterhaltung des Konstrukts in Säugerzellen sorgt.

7. Polynucleotid-Konstrukt nach Anspruch 6, das in Form eines viralen Vektors oder eines Gentargetvektors vorliegt.

8. Polynucleotid-Konstrukt nach Anspruch 7, das ein rekombinanter viraler Vektor ist, der aus Vaccinia, HIV-1, HIV-2, Adenovirus und adenoassoziiertem Virus ausgewählt ist.

9. Polynucleotid-Konstrukt nach Anspruch 7, das ein replikationsdefekter retroviraler Vektor ist.

10. Polynucleotid-Konstrukt nach Anspruch 9, das außerdem eine gp160^{env}-Glycoprotein-codierende Sequenz, die von HIV-1 oder HIV-2 stammt, umfasst.

11. Virales Partikel, das einen viralen Vektor, wie er in einem der Ansprüche 7 bis 10 beansprucht ist, enthält.

12. Virales Partikel nach Anspruch 11, das ein infektiöses replikationsdefektes rekombinantes Retrovirus ist.

13. Zusammensetzung zur Behandlung einer hyperproliferativen Krankheit oder Infektion durch ein infektiöses Agens bei einer Wirtszelle, wobei die Zusammensetzung ein virales Partikel nach Anspruch 11 oder Anspruch 12 und einen pharmazeutisch verträglichen Träger umfasst.

14. Zusammensetzung zur Behandlung einer hyperproliferativen Krankheit oder Infektion durch ein infektiöses Agens, wobei die Zusammensetzung ein Polynucleotid-Konstrukt nach einem der Ansprüche 1 bis 10 und einen pharmazeutisch verträglichen Träger umfasst.

15. Zusammensetzung nach Anspruch 14, wobei der pharmazeutisch verträgliche Träger ein Liposom umfasst.

16. Zusammensetzung nach Anspruch 15, wobei das Liposom außerdem Antikörper umfasst, die für die Wirtszellen, die behandelt werden sollen, spezifisch sind.

17. Konstrukt nach einem der Ansprüche 1 bis 10 oder ein virales Partikel nach Anspruch 11 oder Anspruch 12 zur Verwendung in einem Verfahren zur therapeutischen Behandlung.

18. Verfahren zur Behandlung einer hyperproliferativen Krankheit oder Infektion durch ein infektiöses Agens bei einer ex vivo-Wirtszelle, wobei die Infektion oder die hyperproliferative Krankheit durch einen mit einer humanen Krankheit assoziiertem trans-wirkenden Regulationsfaktor **gekennzeichnet** ist, der fähig ist, die Expression von Genen zu regulieren, wobei das Verfahren Insertieren eines Polynucleotid-Konstrukts nach einem der Ansprüche 1 bis 10 in die Zelle umfasst.

19. Verfahren nach Anspruch 18, wobei die ex vivo-Wirtszelle mit einem viralen Partikel nach Anspruch 11 oder Anspruch 12 infiziert ist.

20. Ex vivo-Zellen, die ein Polynucleotid-Konstrukt nach einem der Ansprüche 1 bis 10 enthalten.

21. Zellen nach Anspruch 20 zur Verwendung in einem Verfahren zur therapeutischen Behandlung.

## Revendications

1. Construction polynucléotidique pour le traitement d'une cellule hôte pour une affection hyperproliférative ou une infection par un agent infectieux, ladite infection ou affection hyperproliférative étant **caractérisée par** un facteur régulateur agissant en trans associé à une maladie humaine, capable de réguler l'expression de gènes, cette construction comprenant :
(i) au moins deux copies en tandem d'une séquence régulatrice agissant en cis qui sont contrôlables par ledit facteur régulateur agissant en trans ; et
(ii) un gène effecteur sous le contrôle de ladite séquence régulatrice agissant en cis, ledit gène effecteur codant une enzyme qui rend ladite cellule sensible à un agent additionnel.

2. Construction polynucléotidique selon la revendication 1, dans laquelle ledit gène effecteur code une nucléoside kinase capable de phosphoryler des analogues de didésoxynucléosides à une vitesse plus élevée que les nucléoside kinases de la cellule hôte.

3. Construction polynucléotidique selon la revendication 2, dans laquelle ledit gène effecteur code une thymidine kinase de virus herpes simplex type 1 (tk de HSV-1).

4. Construction polynucléotidique selon l'une quelconque des revendications 1 à 3, dans laquelle ladite séquence régulatrice agissant en cis est capable de répondre à la protéine tat du HIV.

5. Construction polynucléotidique selon l'une quelconque des revendications 1 à 3, dans laquelle ladite séquence régulatrice agissant en cis est capable de diriger une expression spécifique de cellule.

6. Construction polynucléotidique selon l'une quelconque des revendications 1 à 5, qui comprend en outre une séquence de maintien polynucléotidique qui permet le maintien stable de ladite construction dans des cellules de mammifère.

7. Construction polynucléotidique selon la revendication 6, qui est sous forme d'un vecteur viral ou d'un vecteur cible de gène.

8. Construction polynucléotidique selon la revendication 7 qui est un vecteur viral recombiné choisi parmi la vaccine, HIV-1, HIV-2, l'adénovirus et le virus adéno-associé.

9. Construction polynucléotidique selon la revendication 7 qui est un vecteur rétroviral défectueux pour la réplication.

10. Construction polynucléotidique selon la revendication 9, qui comprend en outre une séquence codant la glycoprotéine gp160^{env} dérivée de HIV-1 ou de HIV-2.

11. Particule virale contenant un vecteur viral selon l'une quelconque des revendications 7 à 10.

12. Particule virale selon la revendication 11, qui est un rétrovirus recombiné infectieux défectueux pour la réplication.

13. Composition pour traiter une cellule hôte pour une affection hyperproliférative ou une infection par un agent infectieux, cette composition comprenant une particule virale selon la revendication 11 ou la revendication 12 et un véhicule pharmaceutiquement acceptable.

14. Composition pour traiter une cellule hôte pour une affection hyperproliférative ou une infection par un agent infectieux, cette composition comprenant une construction polynucléotidique selon l'une quelconque des revendications 1 à 10 et un véhicule pharmaceutiquement acceptable.

15. Composition selon la revendication 14, dans laquelle ledit véhicule pharmaceutiquement acceptable comprend un liposome.

16. Composition selon la revendication 15, dans laquelle ledit liposome comprend en outre des anticorps spécifiques de cellules hôtes désirées à traiter.

17. Construction selon l'une quelconque des revendications 1 à 10 ou particule virale selon la revendication 11 ou la revendication 12, destinée à être utilisée dans une méthode de traitement thérapeutique.

18. Méthode de traitement d'une cellule hôte *ex vivo* pour une affection hyperproliférative ou une infection par un agent infectieux, ladite infection ou affection hyperproliférative étant **caractérisée par** un facteur régulateur agissant en trans associé à une maladie humaine, capable de réguler l'expression de gènes, cette méthode comprenant l'insertion dans ladite cellule d'une construction polynucléotidique selon l'une quelconque des revendications 1 à 10.

19. Méthode selon la revendication 18, dans laquelle ladite cellule hôte *ex vivo* est infectée avec une particule virale selon la revendication 11 ou la revendication 12.

20. Cellules *ex vivo* contenant une construction polynucléotidique selon l'une quelconque des revendications 1 à 10.

21. Cellules selon la revendication 20, destinées à être utilisées dans une méthode de traitement thérapeutique.
